# EUROPÄISCHE PATENTSCHRIFT

(11) **EP 0 758 638 B1**
(45) Veröffentlichungstag und Bekanntmachung des Hinweises auf die Patenterteilung: **30.06.1999**
(21) Anmeldenummer: 96112424.5
(22) Anmeldetag: 01.08.1996
(51) Int. Cl.: C07C 39/17, C07C 39/16

(54) **Adduktkristallisate aus Bisphenol A und Bisphenol TMC**
Cristallisation forms of an adduct of bisphenol-A with bisphenol-TMC
Formes de cristallisation d'un adduit du bisphénol-A et du bisphénol-TMC

(30) Priorität: 14.08.1995 DE 19529857
(43) Veröffentlichungstag der Anmeldung: 19.02.1997
(73) Patentinhaber: BAYER AG, 51368 Leverkusen (DE)
(72) Erfinder: Meurer, Kurt-Peter, Dr., 51375 Leverkusen (DE); Van Osselaer, Tony, Dr., 9100 Belsele (BE); Wulff, Claus, Dr., 47800 Krefeld (DE); Hinz, Jürgen, Dr., 47800 Krefeld (DE)

(56) Entgegenhaltungen:
- DE-A- 2 719 997
- DE-B- 1 168 445

## Beschreibung

Die Erfindung betrifft die Herstellung eines transparenten Adduktkristallisates bestehend aus Bisphenol A (75 Teile) und Bisphenol TMC (1,1-Bis-(4-hydroxyphenyl)-3,3,5-trimethylcyclohexan) (25 Teile), das einen Schmelzpunkt von 144°C besitzt, der unter den Schmelzpunkten der reinen Ausgangskomponenten Bisphenol A (157°C ) und Bisphenol TMC (208°C) liegt.

Verfahrens-, energie- und verarbeitungstechnisch ergeben sich Vorteile bei der Verwendung derartiger Adduktkristalle bei der Herstellung hochtemperaturbeständiger, transparenter und thermostabiler Polymere wie Polycarbonaten.

Bisphenol A (BPA) wird durch Umsetzen von Phenol und Aceton unter sauren Reaktionsbedingungen in Gegenwart von gegebenenfalls modifizierten Ionenaustauscherharzen hergestellt (z.B. DE-A 3 727 641, entsprechend US-A 4 912 263).

Die Isolierung verläuft im wesentlichen über einen abgestuften Kristallisationsschritt der Bisphenol-A-/Phenol-Addukte, anschließende Filtration und Entfernung des überschüssigen Phenols. So hergestelltes Bisphenol-A ist bereits ohne weitere Reinigung als Monomerbaustein zur Herstellung hochtransparenter Polymere, wie z.B. Polycarbonat, geeignet.

Bisphenol TMC wird durch Umsetzen von 3,3,5-Trimethyl-cyclohexanon hergestellt und dient in Mischungen mit Bisphenol A als Zusatzkomponente zur Herstellung von hochtemperaturbeständigen, transparenten Polycarbonaten.

Es wurde nun gefunden, daß eine Mischung aus 75 Teilen hochreinem Bisphenol A und 25 Teilen hochreinem Bisphenol TMC ein Adduktschmelzkristallisat mit einem scharfen Schmelzpunkt bei 144°C bildet.

Dieser Schmelzpunkt liegt unter dem Schmelzpunkt von Bisphenol A (157°C) und weit unter dem des Bisphenol TMC (208°C).

Gegenstand der Erfindung ist die Herstellung eines Adduktkristallisates aus 75 Gew.-Teilen Bisphenol A und 25 Gew.-Teilen Bisphenol TMC mit einem Schmelzpunkt von 144°C, dadurch gekennzeichnet, daß eine Mischung von 90 bis 60 Gew.-Teilen Bisphenol A und 10 bis 40 Gew.-Teilen Bisphenol TMC auf eine Temperatur von 150 bis 160°C erhitzt wird und anschließend auf eine Temperatur < 140°C abgekühlt wird.

Das erfindungsgemäß hergestellte Addukt kann zur Herstellung von hochreinen hochtemperaturbeständigen und transparenten Polymeren wie z.B. bestimmter Polycarbonat-Typen verwendet wird.

Dieses Addukt wird z.B. zur Herstellung hochreiner, transparenter und thermostabiler Schmelzen aus Bisphenol A und Bisphenol TMC bei Temperaturen < 200°C verwendet und kann als Rohstoff bei dieser Temperatur gelagert und verarbeitet werden.

Das erfindungsgemäß hergestellte Addukt kann zur Herstellung eines hochreinen Bisphenol TMC verwendet werden. Dies kann durch ein Destillations- und/oder ein Kristallisationsverfahren erfolgen.

Mischkristallisate aus Bisphenol A und Phenol (60 Teile BPA/40 Teile Phenol) sind bekannt und werden in den Bisphenol-A-Herstellungsverfahren als Prozeßreinigungsschritt eingesetzt.

### Beispiel

75 Teile Bisphenol A und 25 Teile Bisphenol TMC wurden vermischt, bei 160°C geschmolzen und wieder erstarren gelassen. Der Schmelzpunkt des Gemisches wurde bei 144°C festgestellt.

Es wurden DSC-Messungen an Mischungen aus Bisphenol A und Bisphenol TMC durchgeführt (Tab. 1). Bei 144°C wurde ein deutlicher, scharfer Schmelzpunkt bestimmt.

**Tabelle 1**

| GC-Analyse p.p-BPA | TMC-BP | DSC-Analyse | Schmelzbereich | |
|---|---|---|---|---|
| % | % | °C | °C | °C |
| 0,00 | 100 | 205,9 | 203 | 210 |
| 5,00 | 95,0 | 202,4 | 182 | 206 |
| 10,0 | 90,0 | 198,0 | 170 | 205 |
| 15,0 | 85,0 | 196,3 | 140 | 202 |
| 20,0 | 80,0 | 191,7 | 155 | 200 |
| 25,0 | 75,0 | 188,5 | 150 | 198 |
| 30,0 | 70,0 | 184,5 | 145 | 193 |
| 35,0 | 65,0 | 181,5 | 135 | 195 |
| 40,0 | 60,0 | 177,2 | 150 | 195 |
| 45,0 | 55,0 | 173,2 | 133 | 183 |
| 50,0 | 50,0 | 169,3 | 130 | 180 |
| 55,0 | 45,0 | 159,3 | 128 | 180 |
| 60,0 | 40,0 | 141,6 | 115 | 170 |
| 65,0 | 35,0 | 142,8 | 115 | 165 |
| 70,0 | 30,0 | 142,4 | 115 | 165 |
| 75,0 | 25,0 | 144,4 | 115 | 165 |
| 80,0 | 20,0 | 140,2 | 120 | 155 |
| 85,0 | 15,0 | 145,5 | 125 | 155 |
| 90,0 | 10,0 | 152,3 | 125 | 160 |
| 95,0 | 5,00 | 154,8 | 130 | 165 |
| 100 | 0,00 | 157,1 | 145 | 165 |

## Patentansprüche

1. Verfahren zur Herstellung eines Adduktkristallisates aus 75 Gew.-Teilen Bisphenol A und 25 Gew.-Teilen Bisphenol TMC mit einem Schmelzpunkt von 144°C, dadurch gekennzeichnet, daß eine Mischung von 90 bis 60 Gew.-Teilen Bisphenol A und 10 bis 40 Gew.-Teilen Bisphenol TMC auf eine Temperatur von 150 bis 160°C erhitzt wird und anschließend auf eine Temperatur < 140°C abgekühlt wird.

2. Adduktkristallisat aus 75 Gew.-Teilen Bisphenol A und 25 Gew.-Teilen Bisphenol TMC mit einem Schmelzpunkt von 144°C, hergestellt nach Anspruch 1.

## Claims

1. A process for preparing a crystalline adduct of 75 parts by wt. of bisphenol A and 25 parts by wt. of bisphenol TMC with a melting point of 144°C, characterised in that a mixture of 90 to 60 parts by wt. of bisphenol A and 10 to 40 parts by wt. of bisphenol TMC is heated to a temperature of 150 to 160°C and then cooled to a temperature < 140°C.

2. A crystalline adduct of 75 parts by wt. of bisphenol A and 25 parts by wt. of bisphenol TMC with a melting point of 144°C, prepared in accordance with Claim 1.

## Revendications

1. Procédé de préparation d'une forme de cristallisation d'un adduit constitué de 75 parties en poids de bisphénol-A et de 25 parties en poids de bisphénol-TMC, avec un point de fusion de 144°C, caractérisé en ce que l'on chauffe un mélange de 90 à 60 parties en poids de bisphénol-A et de 10 à 40 parties en poids de bisphénol-TMC à une température allant de 150 à 160°C et que l'on refroidit ensuite à une température < 140°C.

2. Forme de cristallisation d'un adduit constitué de 75 parties en poids de bisphénol-A et de 25 parties en poids de bisphénol-TMC, avec un point de fusion de 144°C, préparée suivant la revendication 1.
